# DEMANDE DE BREVET EUROPEEN

(11) **EP 0 727 227 A1**
(43) Date de publication de la demande: **21.08.1996**
(21) Numéro de dépôt: 96400241.4
(22) Date de dépôt: 05.02.1996
(51) Int. Cl.: A61L 2/02, A23L 3/015

(54) **Procédé et dispositif de stérilisation à haute pression de produits**

(30) Priorité: 15.02.1995 FR 9501730
(71) Demandeur: FRAMATOME, 92400 Courbevoie (FR)
(72) Inventeur: Lhenry, Bernard, F-71200 Le Creusot (FR); Plantadis, Rémy, F-71360 Epinac (FR)
(74) Mandataire: Lanceplaine, Jean-Claude

(57) **Abrégé**

La présente invention a pour objet un procédé semi-continu de stérilisation à haute pression d'un produit liquide ou solide dans lequel on effectue la stérilisation de volumes successifs du produit en comprimant à une haute pression ce produit dans une chambre de traitement (2) modulable de forme générale tubulaire et de section circulaire.

L'invention a également pour objet un dispositif de stérilisation à haute pression pour la mise en oeuvre de ce procédé.

## Description

La présente invention a pour objet un procédé et un dispositif semi-continus de stérilisation à haute pression d'un produit liquide ou solide.

Pour stériliser des produits, il est connu de les soumettre à une pression pouvant atteindre une valeur de l'ordre de 8000 bars.

A cet effet, on utilise une enceinte étanche de volume réduit dans laquelle sont placés les produits à stériliser qui sont soumis à une haute pression. Cette enceinte étanche est raccordée à une pompe destinée à gonfler l'intérieur de l'enceinte dans le but d'augmenter la pression interne.

Dans le cas d'un fluide à stériliser, ce fluide est contenu dans une enceinte étanche et soumis directement à la pression désirée obtenue à l'aide de la pompe.

Dans le cadre d'un élément emballé à stériliser, cet élément est placé dans l'enceinte étanche et est soumis à la pression requise au moyen de ladite pompe.

Mais, les dispositifs utilisés jusqu'à présent ne permettent pas de stériliser des quantités importantes de produits.

L'invention a pour but de proposer un procédé et un dispositif semi-continus de stérilisation à haute pression d'un produit liquide ou solide permettant de stériliser des quantités importantes de produits et présentant toutes les garanties nécessaires de sécurité, tout en conservant au produit stérilisé ses qualités originelles, comme par exemple son goût et son aspect.

L'invention a pour objet un procédé semi-continu de stérilisation à haute pression d'un produit liquide ou solide, caractérisé en ce que l'on effectue la stérilisation de volumes successifs d'un produit dans une chambre de traitement modulable de forme générale tubulaire et de section circulaire, comportant deux extrémités opposées et ouvertes, en réalisant les étapes suivantes :
- on introduit au niveau de la première extrémité de la chambre, un premier séparateur de section correspondant à la section de la chambre,
- on obture la seconde extrémité de la chambre,
- on injecte dans la chambre, au niveau de la première extrémité, un premier volume du produit à stériliser de façon à remplir ladite chambre et à déplacer le premier séparateur jusqu'à la seconde extrémité,
- on introduit dans la chambre, au niveau de la première extrémité, un second séparateur de section correspondant à la section de la chambre,
- on comprime le premier volume du produit jusqu'à une pression désirée pour stériliser ce premier volume du produit,
- on ouvre la seconde extrémité de la chambre,
- on évacue de la chambre le premier séparateur et le premier volume du produit stérilisé par l'injection au niveau de la première extrémité de ladite chambre d'un second volume du produit à stériliser,
- on récupère le premier séparateur et le premier volume du produit stérilisé,
- on stérilise la première extrémité de la chambre après chaque injection de volume du produit,
- on stérilise un sas de récupération des séparateurs après chaque récupération de volume du produit stérilisé,
- on nettoie le premier séparateur,
- et, après la fermeture de la seconde extrémité de la chambre et l'injection dans ladite chambre du second volume du produit à stériliser, on introduit dans la chambre au niveau de la première extrémité, le premier séparateur et on renouvelle le cycle pour la totalité du produit à stériliser.

L'invention a également pour objet un dispositif de stérilisation semi-continu à haute pression d'un produit liquide ou solide, caractérisé en ce qu'il comprend :
- un tube de longueur modulable formant une chambre interne de traitement, de section circulaire et comportant deux extrémités ouvertes et opposées,
- des moyens d'injection dans la chambre de volumes successifs du produit à stériliser, disposés au niveau de la première extrémité de ladite chambre,
- des moyens d'obturation de la seconde extrémité de la chambre,
- des moyens de compression des volumes successifs du produit pour stériliser ledit produit,
- des moyens de séparation du volume du produit stérilisé avec le volume du produit à stériliser,
- des moyens de stérilisation de la première extrémité de la chambre,
- des moyens de récupération des volumes successifs du produit stérilisé, disposés au niveau de la deuxième extrémité de la chambre,
- des sas de récupération des moyens de séparation,
- des moyens de stérilisation du sas de récupération situé à proximité de la seconde extrémité de la chambre,
- et des moyens de transfert des moyens de séparation d'une extrémité à l'autre de la chambre interne de traitement.

Selon d'autres caractéristiques de l'invention :
- le tube de longueur modulable est formé par plusieurs éléments tubulaires indépendants fixés bout à bout au moyen de brides de raccordement boulonnées, un joint d'étanchéité étant disposé au niveau de l'interface des éléments tubulaires associés,
- les moyens d'injection dans la chambre de volumes successifs du produit à stériliser sont formés par une vanne à tiroir de remplissage comportant, à sa partie inférieure, un orifice transversal et, à sa partie supérieure, un canal de circulation du produit à stériliser communiquant avec un réservoir d'alimentation sous pression par un tuyau télescopique, ladite vanne de remplissage étant déplaçable verticalement par un organe de commande entre une première position mettant en communication l'orifice transversal avec la chambre et une seconde position mettant en communication le canal de circulation avec ladite chambre,
- les moyens d'obturation de la seconde extrémité de la chambre comprennent, d'une part, un piston de section circulaire, déplaçable dans l'axe de ladite chambre et monté à l'extrémité d'une tige d'un vérin et, d'autre part, un moyen de verrouillage du piston en position d'obturation de la chambre,
- les moyens de compression des volumes successifs du produit sont formés par un piston de section circulaire et déplaçable dans l'axe de la chambre pour pénétrer au niveau de la première extrémité de ladite chambre, ledit piston étant fixé à l'extrémité libre d'une tige d'un vérin,
- les moyens de récupération des volumes successifs du produit stérilisé sont formés par une vanne à tiroir de vidange comportant, à sa partie inférieure, un orifice transversal et, à sa partie supérieure, un canal de circulation du produit stérilisé communiquant avec un réservoir de récupération stérile par un tuyau télescopique, ladite vanne de récupération étant déplaçable verticalement par un organe de commande entre une première position mettant en communication l'orifice transversal avec la chambre et une seconde position mettant en communication le canal de circulation avec ladite chambre,
- les moyens de séparation du volume du produit stérilisé avec le volume du produit à stériliser sont formés par des séparateurs cylindriques de diamètre correspondant au diamètre de la chambre, destinés à être déplacés dans la chambre entre chaque volume de produit et comportant des organes d'étanchéité avec la paroi interne de ladite chambre,
- les moyens de stérilisation de la première extrémité de la chambre sont formés par un canal ménagé dans le piston et débouchant au niveau de l'extrémité libre dudit piston, ledit canal étant relié par un orifice à des moyens d'injection de vapeur,
- les moyens de transfert des séparateurs cylindriques sont formés par un tuyau de retour s'étendant d'une extrémité à l'autre du tube et comportant à une de ses extrémités le sas relié à la seconde extrémité de la chambre par la vanne à tiroir de vidange et à l'autre de ses extrémités le sas relié à la première extrémité de la chambre par la vanne à tiroir de remplissage.

Les caractéristiques et avantages de l'invention apparaîtront au cours de la description qui va suivre, donnée uniquement à titre d'exemple, faite en référence aux dessins annexés, sur lesquels :
- la Fig. 1 est une vue schématique en élévation d'un dispositif de stérilisation à haute pression d'un produit, selon l'invention,
- la Fig. 2 est une vue schématique en coupe des moyens d'injection du produit et de mise en compression de ce produit dans la chambre de traitement du dispositif de stérilisation selon l'invention,
- la Fig. 3 est une vue en coupe et à plus grande échelle des moyens d'injection du produit et de mise en compression de ce produit,
- la Fig. 4 est une vue en coupe selon la ligne 4-4 de la Fig. 2,
- la Fig. 5 est une vue schématique en coupe et à plus grande échelle des moyens de récupération du produit stérilisé et des moyens de transfert des séparateurs cylindriques de séparation du dispositif de stérilisation selon l'invention,
- la Fig. 6 est une vue en coupe selon la ligne 6-6 de la Fig. 5,
- les Figs. 7A à 7H sont des schémas montrant les différentes phases de fonctionnement du dispositif de stérilisation selon l'invention.

Selon les figures, on a représenté un dispositif semi-continu de stérilisation à haute pression d'un produit liquide ou solide, comme par exemple des fluides ou des aliments.

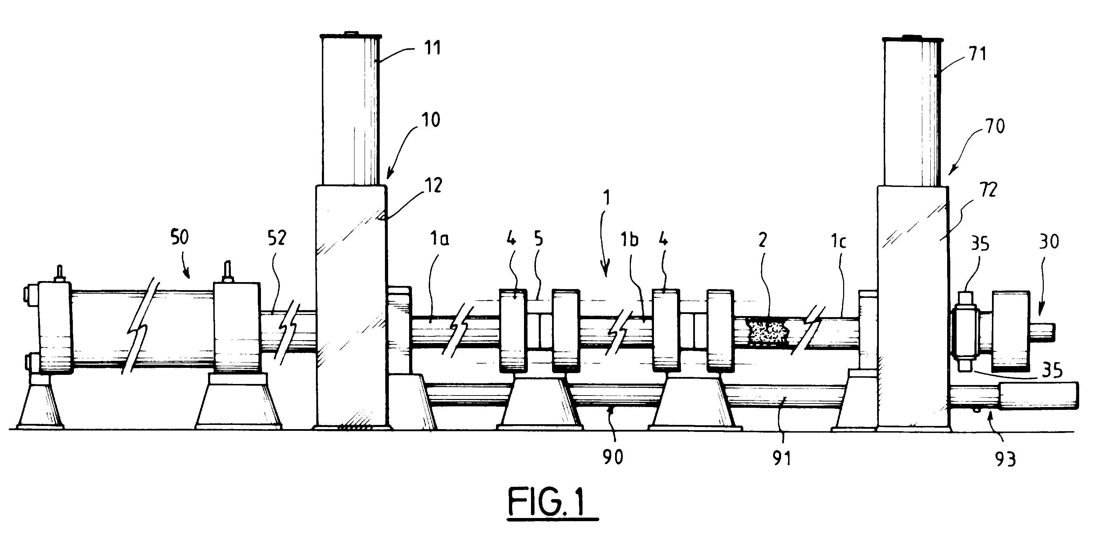

Le dispositif de stérilisation représenté à la Fig. 1 comprend :
- un tube 1 de longueur modulable formant une chambre interne 2 de traitement, de section circulaire et comportant deux extrémités 2a et 2b ouvertes et opposées,
- des moyens 10 d'injection dans la chambre de volumes successifs du produit à stériliser, disposés au niveau de la première extrémité 2a de la chambre 2,
- des moyens 30 d'obturation de la seconde extrémité 2b de la chambre 2,
- des moyens 50 de compression des volumes successifs du produit pour stériliser ledit produit,
- des moyens 3 de séparation du volume du produit stérilisé avec le volume du produit à stériliser,
- des moyens 25 et 26 de stérilisation de la première extrémité 2a de la chambre 2,
- des moyens 70 de récupération des volumes successifs du produit stérilisé, disposés au niveau de la deuxième extrémité 2b de la chambre 2,
- de sas 92 et 110 de récupération des moyens de séparation 3,
- des moyens 85 de stérilisation du sas 92 de récupération,
- et des moyens 90 de transfert des moyens de séparation 3 d'une extrémité à l'autre de la chambre 2.

Comme représenté à la Fig. 1, le tube 1 de longueur modulable est formé de plusieurs éléments tubulaires 1a, 1b, 1c..., indépendants et fixés bout à bout au moyen de brides de raccordement 4 fixées entre elles par exemple par des boulons 5.

Un joint d'étanchéité, non représenté, est disposé au niveau de l'interface des éléments tubulaires 1a, 1b, 1c... associés.

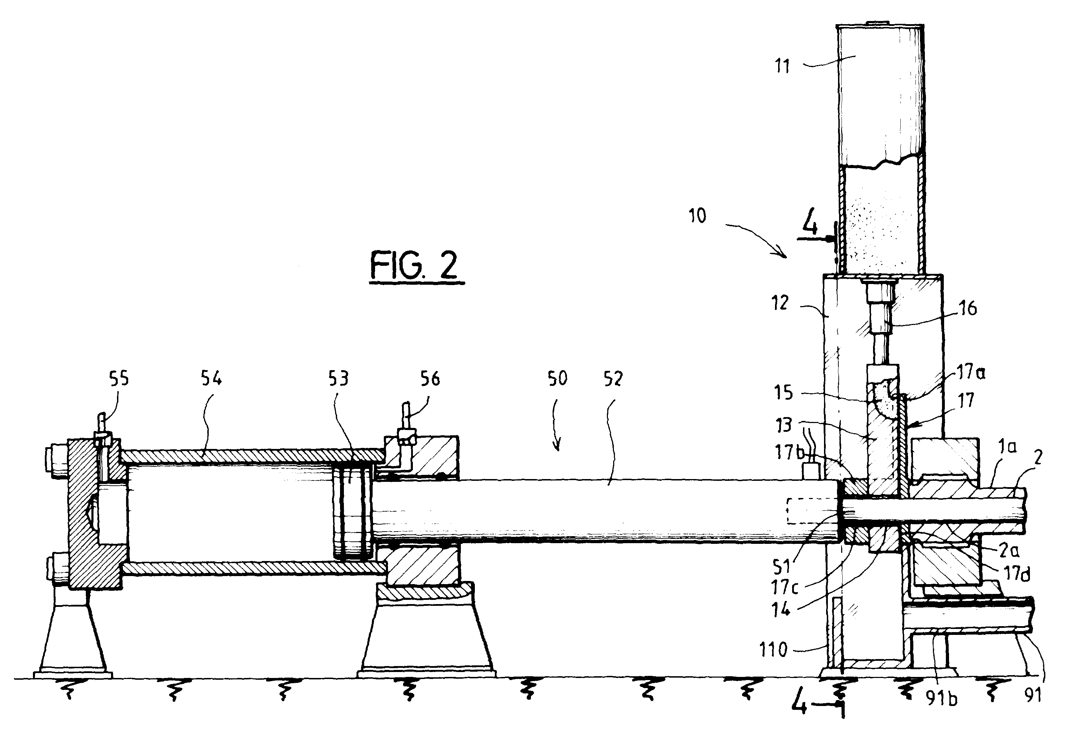

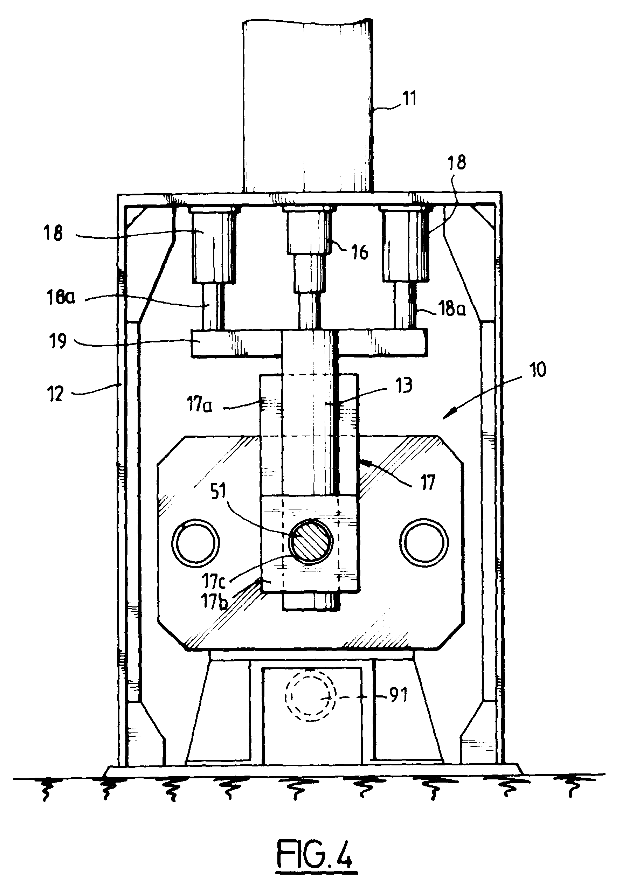

En se reportant aux Figs. 2 à 4, on va maintenant décrire les moyens 10 d'injection dans la chambre de traitement 2 du produit à stériliser.

Ces moyens 10 d'injection sont constitués par un réservoir d'alimentation 11 sous pression contenant le produit à stériliser et fixé sur un socle 12.

Les moyens 10 d'injection comprennent également une vanne à tiroir 13 de remplissage formée par un plaque de section rectangulaire comportant, à sa partie inférieure, un orifice transversal 14 et, à sa partie supérieure, un canal 15 de circulation du produit à stériliser communiquant avec le réservoir 11 d'alimentation sous pression par un tuyau télescopique 16.

La vanne de remplissage 13 est déplaçable verticalement par un organe de commande entre une première position mettant en communication l'orifice transversal 14 avec la chambre 2 et une seconde position mettant en communication le canal de circulation 15 avec ladite chambre 2.

Pour cela, la vanne à tiroir 13 est, au cours de son déplacement vertical, guidée par une pièce 17 fixée sur l'extrémité 2a du tube 1a.

La pièce 17 est formée par une plaque verticale 17a sur laquelle coulisse la vanne à tiroir 13 et par un fourreau 17b comportant, d'une part, un alésage horizontal 17c disposé dans l'axe de la chambre 2 et de diamètre correspondant au diamètre de ladite chambre 2 et, d'autre part, un passage vertical 17d de section correspondant à la section de la vanne à tiroir 13 et destiné au coulissement de ladite vanne à tiroir 13.

L'organe de commande du déplacement de la vanne à tiroir 13 est constitué d'au moins un verin 18 et de préférence de deux vérins 18 verticaux et parallèles, supportés par le socle 12. La tige 18a de chaque vérin 18 est solidaire d'une embase 19 fixée à la partie supérieure de ladite vanne à tiroir 13.

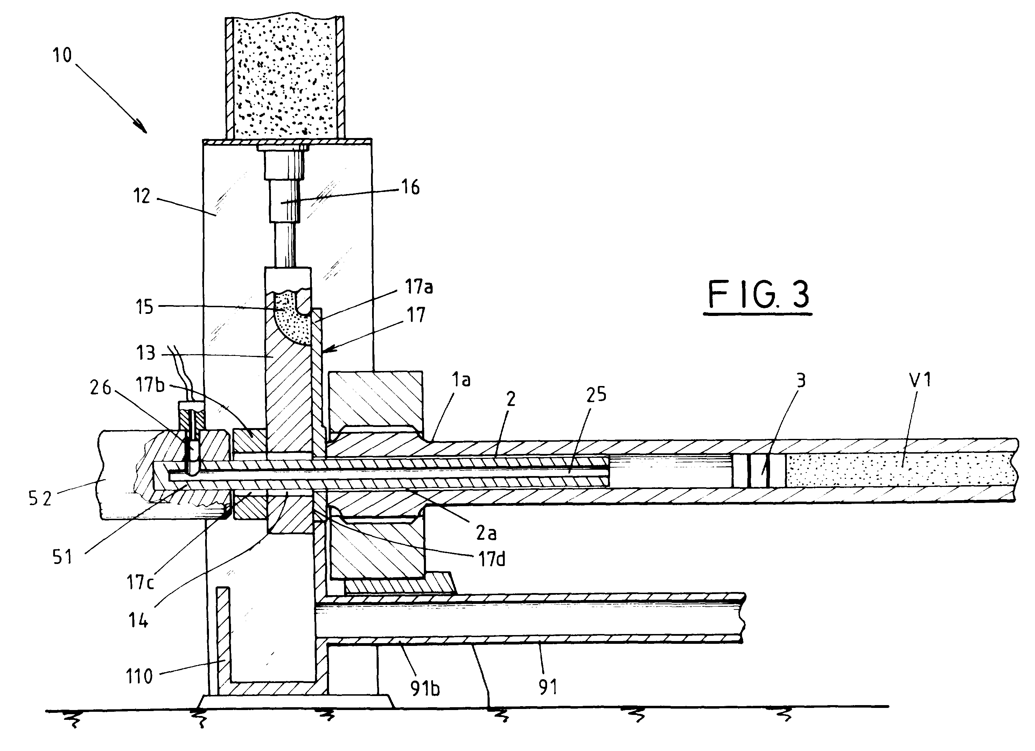

Le dispositif comporte également des moyens de stérilisation de la première extrémité 2a de la chambre de traitement 2 qui sont représentés à la fig. 3.

Ces moyens de stérilisation sont formés par un canal 25 ménagé dans le piston 51 et débouchant au niveau de l'extrémité libre dudit piston 51. Le canal 25 est relié par un orifice 26 à des moyens d'injection de vapeur, non représentés, de façon à stériliser l'extrémité 2a de la chambre 2, comme on le verra ultérieurement.

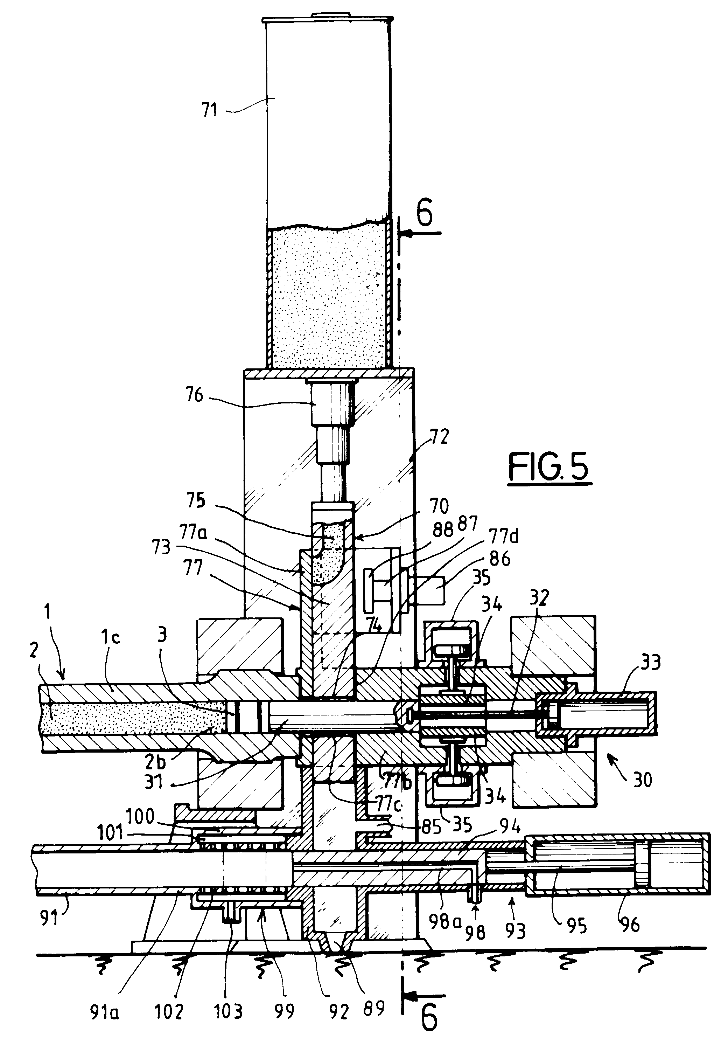

Ainsi que représenté à la Fig. 5, les moyens 30 d'obturation de la seconde extrémité 2b de la chambre de traitement 2 comprennent un piston 31 de section circulaire et monté à l'extrémité d'une tige 32 d'un vérin 33.

Le piston 31 est déplaçable horizontalement entre une première position dans laquelle l'extrémité libre dudit piston 31 est placée dans la chambre de traitement 2 et une seconde position dans laquelle ladite extrémité libre du piston 31 est située en dehors de ladite chambre 2.

Le piston 31 est associé à des moyens de verrouillage dudit piston 31 en position d'obturation de la chambre 2, qui sont constitués par deux mâchoires 34 opposées et commandées chacune par un vérin 35.

Les mâchoires 34 sont déplaçables verticalement entre une position de blocage du piston 31 comme représentée à la Fig. 5 et une position de déverrouillage libérant ledit piston 31.

Comme représenté aux Figs. 2 et 3, les moyens 50 de compression du produit dans la chambre de traitement 2 sont constitués par un piston 51 de section circulaire et de diamètre correspondant au diamètre de la chambre de traitement 2.

Le piston 51 est fixé à l'extrémité libre d'une tige 52 comportant à son extrémité opposée à celle sur laquelle est fixée le piston 51, un piston de commande 53 d'un vérin 54.

De manière classique, le vérin 54 comporte des conduits 55 et 56 d'alimentation en fluide sous pression.

Le piston 51 est déplaçable horizontalement dans l'axe de la chambre de traitement 2 entre une première position dans laquelle l'extrémité libre de ce piston 51 est introduite dans la chambre de traitement 2 en passant par l'orifice transversal 14 de la vanne à tiroir 13, comme représenté à la Fig. 2 et une seconde position dans laquelle l'extrémité libre dudit piston 51 est rétractée et positionnée en dehors de la chambre 2 et de l'alésage horizontal 14 de la vanne à tiroir 13.

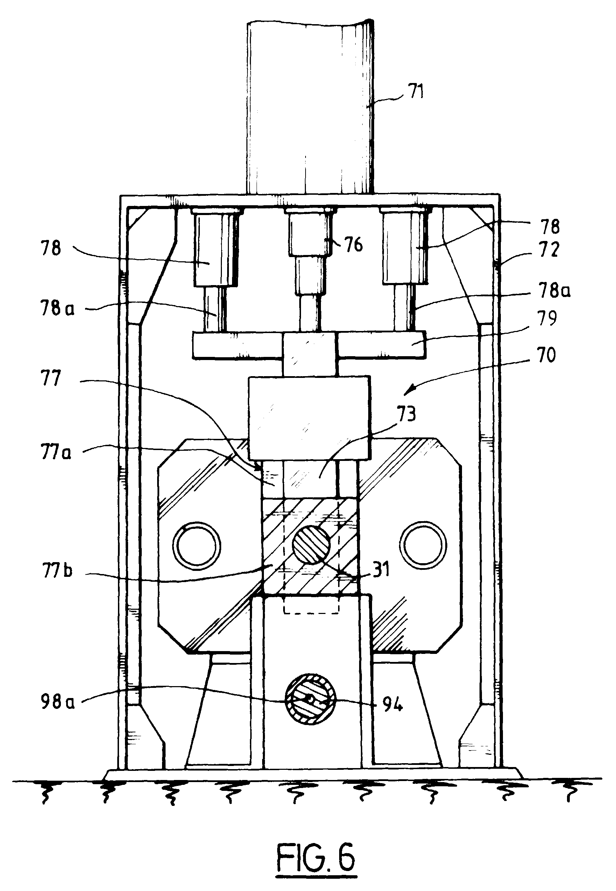

Comme cela apparait sur les Figs. 5 et 6, les moyens 70 de récupération du produit stérilisé sont formés par un réservoir 71 de récupération stérile, supporté par un socle 72.

Les moyens 70 de récupération comprennent également une vanne à tiroir 73 de vidange formée par une plaque de section rectangulaire comportant, à sa partie inférieure, un orifice transversal 74 pour le passage du piston d'obturation 31 et, à sa partie supérieure, un canal de circulation 75 communiquant avec le réservoir de récupération 71 par un tuyau télescopique 76.

La vanne à tiroir 73 de récupération est déplaçable verticalement par un organe de commande entre une première position mettant en communication l'orifice transversal 74 avec la chambre 2 et une seconde position mettant en communication le canal de circulation 75 avec ladite chambre 2.

Pour cela, la vanne à tiroir 73 est, au cours de son déplacement vertical, guidée par une pièce 77 fixée sur l'extrémité 2b du tube 1c.

La pièce 77 est formée par une plaque verticale 77a sur laquelle coulisse la vanne à tiroir 73 et par un fourreau 77b comportant un alésage horizontal 77c disposé dans l'axe de la chambre de traitement 2 et un passage vertical 77d de section correspondant à la section de la vanne à tiroir 73 et destiné à permettre le coulissement vertical de la vanne à tiroir 73 entre les deux positions.

Comme représenté à la Fig. 6, l'organe de commande du déplacement de la vanne à tiroir 73 est constitué d'au moins un verin 78 et de préférence de deux vérins 78 verticaux, supportés par le socle 72.

La tige 78a de chaque vérin 78 est solidaire d'une embase 79 fixée à la partie supérieure de ladite vanne à tiroir 73.

Le fourreau 77b de la pièce 77 sert d'organe de guidage du piston 31 et d'organe de support du vérin 33 et des deux vérins 35 de commande des mâchoires 34.

Le dispositif de stérilisation tel que décrit ci-dessus permet de stériliser des volumes successifs de produit et comporte, à cet effet, des moyens 3 de séparation du volume du produit stérilisé avec le volume du produit à stériliser.

Ces moyens de séparation sont formés par plusieurs séparateurs cylindriques indépendants de diamètre correspondant au diamètre de la chambre 2 et comportant des organes d'étanchéité avec la paroi de ladite chambre 2.

Ces séparateurs cylindriques 3 sont destinés à être déplacés de l'extrémité 2a vers l'extrémité 2b de la chambre de traitement 2 entre chaque volume de produit et à être recyclés de la seconde extrémité 2b vers la première extrémité 2a de ladite chambre de traitement 2.

A cet effet, le dispositif comporte des moyens 90 de transfert des séparateurs cylindriques 3 d'une extrémité à l'autre de la chambre 2.

En se reportant aux figures 1, 2 et 5, on va décrire ces moyens 90 de transfert des séparateurs cylindriques 3.

Les moyens 90 de transfert des séparateurs cylindriques sont formés par un tuyau de retour 91 disposé au-dessous du tube 1 et s'étendant sur toute la longueur dudit tube 1.

Le tuyau de retour 91 comporte à son extrémité 91a située au-dessous de l'extrémité 2b du tube 2, un sas 92 relié à ladite extrémité 2b du tube 1 par la vanne à tiroir 73 de vidange.

Le dispositif comporte des moyens de stérilisation du sas 92 de récupération des séparateurs 3. Ces moyens sont formés par un orifice 85 ménagé dans une paroi dudit sas 92 et relié à des moyens d'injection de vapeur, non représentés.

Pendant la stérilisation du sas 92 par injection de vapeur par l'orifice 85, la vanne à tiroir 73 de vidange est plaquée contre la plaque verticale 77a de façon à assurer l'étanchéité du canal de circulation 75.

Pour cela, le socle 72 supporte un vérin 86 comportant une tige 87 dont l'extrémité libre est pourvue d'une plaque 88 destinée à venir s'appuyer sur la vanne à tiroir 73 pour appliquer ladite vanne à tiroir 73 sur la plaque verticale 77a lors de la stérilisation du sas 92.

De plus, le fond du sas 92 est équipé d'un orifice 89 d'évacuation des saletés pendant la stérilisation du sas 92.

Le tuyau 91 comprend également à son extrémité 91a, un organe 93 d'introduction des séparateurs cylindriques 3 dans le tuyau de retour 91, des moyens 98 de propulsion des séparateurs cylindriques 3 dans le tuyau de retour 91 et des moyens 99 de nettoyage desdits séparateurs cylindriques 3.

L'organe 93 d'introduction des séparateurs cylindriques 3 dans le tuyau de retour 91 est formé par un piston 94 fixé à l'extrémité libre d'une tige 95 d'un vérin 96.

Le piston 94 est déplaçable horizontalement entre une première position dans laquelle son extrémité libre pénètre dans le tuyau de retour 91 en traversant le sas 92 et une seconde position dans laquelle ladite extrémité libre du piston 94 est située en dehors du sas 92.

Les moyens 98 de propulsion des séparateurs cylindriques 3 dans le tuyau de retour 91 sont formés par un canal 98a qui traverse sur toute sa longueur le piston 94 et qui débouche au niveau de l'extrémité libre dudit piston 94.

Ce conduit 98a est relié à une source d'alimentation, non représentée, d'un fluide sous pression.

Les moyens 99 de nettoyage comprennent un manchon 100 disposé autour de l'extrémité 91a et qui détermine une chambre 101 dans laquelle le tuyau de retour 91 est percé d'une multitude de petits orifices 102.

La chambre 101 est raccordée par un conduit 103 à des moyens, non représentés, d'alimentation d'un fluide de nettoyage et de stérilisation des séparateurs cylindriques 3.

L'extrémité 91b du tuyau de retour 91 située au-dessous de l'extrémité 2a de la chambre 2 du tube 1 comporte un sas 110 de récupération des séparateurs cylindriques 3 et qui communique avec l'extrémité 2a de la chambre 2 par la vanne à tiroir 13 pour le transfert desdits séparateurs cylindriques du tuyau de retour 91 dans la chambre de traitement 2.

Le sas 110 sert également de chambre de récupération de l'eau lors de l'injection de vapeur d'eau au niveau de l'extrémité 2a de la chambre 2.

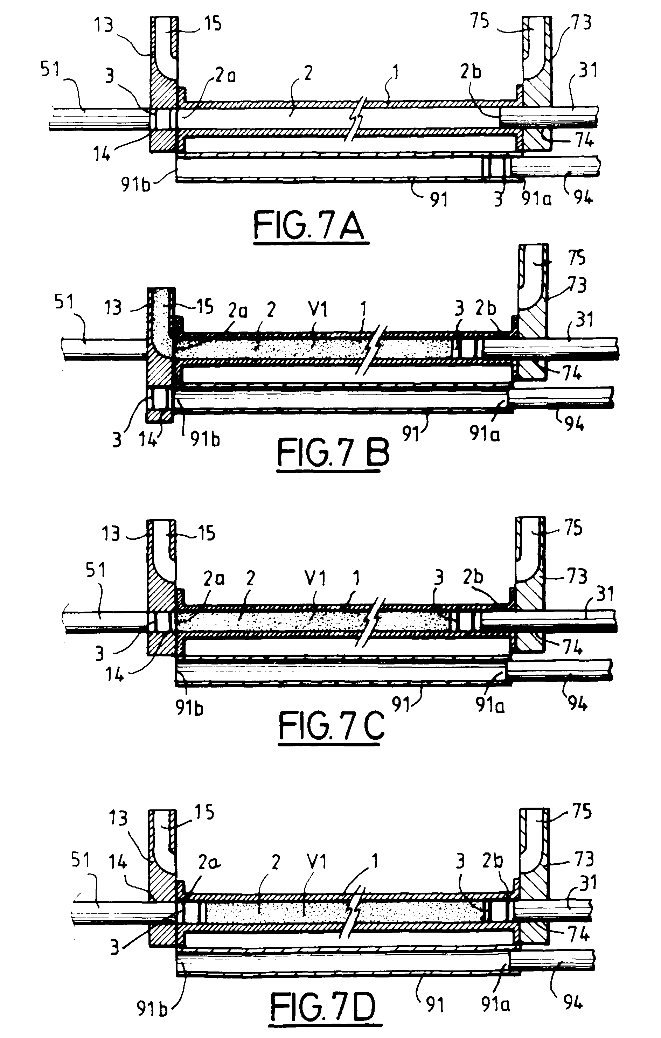

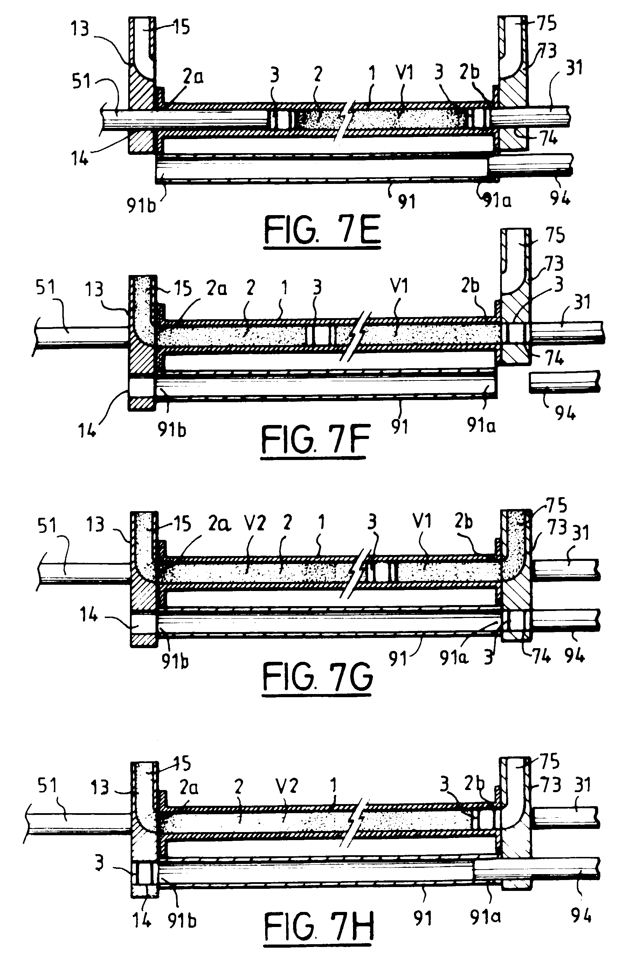

En se reportant maintenant aux figures 7A à 7H, on va décrire le fonctionnement du dispositif de stérilisation selon l'invention.

Tout d'abord et comme représenté à la Fig. 7A, la vanne à tiroir 13 d'alimentation est en position haute pour que l'orifice transversal 14 dans lequel a été placé un séparateur cylindrique 3, soit positionné dans l'alignement de la chambre 2.

La vanne à tiroir 73 de récupération est également en position haute et l'extrémité 2b de la chambre 2 est obturée par le piston 31 qui traverse l'orifice transversal 74 de ladite vanne à tiroir 73.

Un second séparateur cylindrique 3 est en attente dans l'extrémité 91a du tuyau de retour 91.

Le piston 51 du vérin 54 est déplacé pour introduire le premier séparateur cylindrique 3 dans la chambre de traitement 2.

Ensuite, le piston 51 est ramené en position initiale et la vanne à tiroir 13 d'alimentation est amenée en position basse pour mettre en communication le réservoir d'alimentation 11 avec la chambre de traitement 2 par l'intermédiaire du tuyau télescopique 16 et du canal de circulation 15, comme représenté à la Fig. 7B.

Un premier volume V1 de produit à stériliser est injecté dans la chambre 2 ce qui déplace le premier séparateur cylindrique 3 jusqu'au piston 31.

Simultanément, le fluide sous pression est injecté dans le tuyau de retour 91 par le conduit 98a du piston 94 ce qui a pour effet de déplacer le second séparateur cylindrique 3 dans ce tuyau de retour 91 et de placer ledit second séparateur cylindrique 3 dans l'orifice transversal 14 de la vanne à tiroir 13.

Lorsque le premier volume V1 du produit à stériliser a été injecté dans la chambre de traitement 2, la vanne à tiroir 13 d'alimentation est placée en position haute et le piston 51 du vérin 54 est déplacé horizontalement en direction de la chambre de traitement 2 afin d'introduire le second séparateur cylindrique 3 dans cette chambre de traitement et de comprimer à une pression déterminée le premier volume V1 du produit (Figs. 7D et 7E).

Dès que le premier volume V1 du produit a été comprimé et donc stérilisé, le piston 51 de mise en compression est ramené en dehors de la chambre de traitement 2, la vanne à tiroir 13 de remplissage est placée en position basse de façon à mettre de nouveau le réservoir 11 en communication avec cette chambre de traitement 2 par le tuyau télescopique 16 et le canal d'alimentation 15, puis le piston d'obturation 31 est ramené en dehors de la chambre de traitement 2 ainsi que représenté à la Fig. 7F.

Au cours du retour du piston 51 dans sa position initiale, de la vapeur d'eau est injectée par l'orifice 26 et le canal 25, le volume V1 du produit et le second séparateur 3 formant un bouchon étanche.

La vapeur d'eau circule dans le canal 25, puis pénètre dans l'extrémité 2a de la chambre 2 et retourne par le jeu fonctionnel existant entre le piston 51 et la chambre 2 ce qui a pour effet de stériliser l'extrémité 2a de ladite chambre 2 avant l'injection d'un nouveau volume du produit.

L'eau est récupérée dans le sas 110.

Cette stérilisation est effectuée entre chaque injection du produit à stériliser et l'injection de vapeur d'eau est maintenue pendant le temps nécessaire afin d'obtenir la stérilisation adéquate.

La vanne à tiroir 73 de récupération est placée en position basse pour mettre en communication la chambre de traitement 2 avec le réservoir de récupération 71 stérile et positionner le premier séparateur cylindrique 3 dans l'alignement du tuyau de retour 91, comme cela apparaît sur la Fig. 7G.

Ensuite, un second volume V2 du produit à traiter est injecté dans la chambre de traitement 2 à partir du réservoir d'alimentation 11 provoquant ainsi le transfert du premier volume V1 du produit stérilisé dans le réservoir de récupération 71 et le déplacement du second séparateur cylindrique 3 jusqu'à l'extrémité 2b de la chambre de traitement 2.

De plus, entre chaque récupération du produit stérilisé, de la vapeur d'eau est injectée par l'orifice 85 pour stériliser le sas 92 et récupérer les saletés par l'orifice 89.

Au cours de cette injection de vapeur d'eau, le vérin 86 est actionné pour que, sous l'effet de la poussée exercée par la tige 87, la plaque 88 vienne s'appuyer sur la vanne à tiroir 73.

Ainsi la vanne à tiroir 73 est plaquée contre la plaque verticale 77a et l'étanchéité du canal de circulation 75 est assurée.

Simultanément, le piston 94 est déplacé horizontalement pour transférer le second séparateur cylindrique 3 du sas 92 dans la chambre de nettoyage 101 dans laquelle est injecté un fluide de nettoyage et de stérilisation.

Une fois le second séparateur cylindrique 3 nettoyé et stérilisé, le fluide sous pression est injecté par le conduit 98a du piston 94 pour déplacer ce second séparateur cylindrique 3 dans le tuyau de retour 91 et le placer en attente dans l'orifice transversal 14 de la vanne à tiroir 13.

Lorsque le second volume V2 de produit est stérilisé, le cycle recommence et ainsi de suite jusqu'à la stérilisation de la totalité du produit.

Chaque volume du produit est comprimé à une pression comprise entre 5000 et 8000 bars.

Grâce à l'utilisation d'une chambre de traitement modulable, le dispositif de stérilisation à haute pression selon l'invention permet de stériliser des volumes importants de produit, comme par exemple des produits liquides ou solides, tout en présentant toutes les garanties de sécurité indispensables à l'utilisation d'une très haute pression.

De plus, le procédé et le dispositif de stérilisation selon l'invention permettent au produit stérilisé de conserver ces qualités originelles, comme par exemple son goût et son aspect.

## Revendications

1. Procédé semi-continu de stérilisation à haute pression d'un produit liquide ou solide, caractérisé en ce que l'on effectue la stérilisation de volumes successifs du produit dans une chambre de traitement (2) modulable de forme générale tubulaire et de section circulaire, comportant deux extrémités (2a, 2b) opposées et ouvertes en réalisant les étapes suivantes :
- on introduit, au niveau de la première extrémité (2a) de la chambre (2), un premier séparateur (3) de section correspondant à la section de la chambre (2),
- on obture la seconde extrémité (2b) de la chambre (2),
- on injecte dans la chambre (2), au niveau de la première extrémité (2a), un premier volume du produit à stériliser de façon à remplir ladite chambre et à déplacer le premier séparateur (3) jusqu'à la seconde extrémité (2b),
- on introduit dans la chambre (2), au niveau de la première extrémité (2a), un second séparateur (3) de section correspondant à la section de la chambre (2),
- on comprime le premier volume du produit jusqu'à une pression désirée pour stériliser ce premier volume du produit,
- on ouvre la seconde extrémité (2b) de la chambre (2),
- on évacue de la chambre (2) le premier séparateur (3) et le premier volume du produit stérilisé par l'injection, au niveau de la première extrémité (2b) de ladite chambre (2), d'un second volume du produit à stériliser,
- on récupère le premier séparateur (3) et le premier volume du produit stérilisé,
- on stérilise la première extrémité (2a) de la chambre (2) après chaque injection de volume du produit,
- on stérilise un sas de récupération des séparateurs (3) après chaque récupération de volume du produit stérilisé,
- on nettoie le premier séparateur (3),
- et, après la fermeture de la seconde extrémité (2b) de la chambre (2) et l'injection dans ladite chambre (2) du second volume du produit à stériliser, on introduit dans la chambre (2) au niveau de la première extrémité (2a) le premier séparateur (3) et on renouvelle le cycle pour la totalité du produit à stériliser.

2. Procédé selon la revendication 1, caractérisé en ce que l'on comprime successivement chaque volume du produit à une pression comprise entre 5000 et 8000 bars.

3. Dispositif de stérilisation en semi-continu à haute pression d'un produit liquide ou solide, caractérisé en ce qu'il comprend :
- un tube (1) de longueur modulable formant une chambre interne (2) de traitement, de section circulaire et comportant deux extrémités (2a, 2b) ouvertes et opposées,
- des moyens (10) d'injection dans la chambre (2) de volumes successifs du produit à stériliser, disposés au niveau de la première extrémité (2a) de ladite chambre (2),
- des moyens (30) d'obturation de la seconde extrémité (2b) de la chambre (2),
- des moyens (50) de compression des volumes successifs du produit pour stériliser ledit produit,
- des moyens (3) de séparation du volume du produit stérilisé avec le volume du produit à stériliser,
- des moyens (25, 26) de stérilisation de la première extrémité (2a) de la chambre (2),
- des moyens (70) de récupération des volumes successifs du produit stérilisé, disposés au niveau de la deuxième extrémité (2b) de la chambre (2),
- des sas (92, 110) de récupération des moyens de séparation (3),
- des moyens (85) de stérilisation du sas (92) de récupération, situé à proximité de la seconde extrémité (2b) de la chambre (2),
- et des moyens (90) de transfert des moyens de séparation (3) d'une extrémité à l'autre de la chambre interne de traitement (2).

4. Dispositif selon la revendication 3, caractérisé en ce que le tube (1) de longueur modulable est formé de plusieurs éléments (1a, 1b, 1c) tubulaires et indépendants, fixés bout à bout au moyen de brides de raccordement (4) boulonnées, un joint d'étanchéité étant disposé au niveau de l'interface des éléments tubulaires associés.

5. Dispositif selon la revendication 3, caractérisé en ce que les moyens (10) d'injection dans la chambre (2) de volumes successifs de produit à stériliser sont formés par une vanne à tiroir (13) de remplissage comportant, à sa partie inférieure, un orifice transversal (14) et, à sa partie supérieure, un canal (15) de circulation du produit à stériliser communiquant avec un réservoir (11) d'alimentation sous pression par un tuyau télescopique (16), ladite vanne à tiroir (13) de remplissage étant déplaçable verticalement par un organe de commande (18) entre une première position mettant en communication l'orifice transversal (14) avec la chambre (2) et une seconde position mettant en communication le canal de circulation (15) avec ladite chambre (2).

6. Dispositif selon la revendication 5, caractérisé en ce que la vanne à tiroir (13) de remplissage est formée par une plaque de section rectangulaire.

7. Dispositif selon les revendications 5 et 6, caractérisé en ce que l'organe de commande du déplacement de la vanne à tiroir (13) de remplissage est constitué par au moins un vérin (18).

8. Dispositif selon la revendication 3, caractérisé en ce que les moyens (30) d'obturation de la seconde extrémité (2b) de la chambre (2) comprennent, d'une part, un piston (31) de section circulaire, déplaçable dans l'axe de ladite chambre (2) et monté à une extrémité d'une tige (32) d'un vérin (33) et, d'autre part, un moyen (34, 35) de verrouillage du piston (31) en position d'obturation de la chambre (2).

9. Dispositif selon la revendication 8, caractérisé en ce que le moyen de verrouillage comprend deux mâchoires (34) opposées, commandées chacune par un vérin (35) et déplaçables entre une position de blocage du piston d'obturation (31) et une position de déverrouillage dudit piston (31).

10. Dispositif selon la revendication 3, caractérisé en ce que les moyens (50) de compression des volumes successifs du produit sont formés par une piston (51) de section circulaire et déplaçable dans l'axe de la chambre (2) pour pénétrer au niveau de la première extrémité (2a) de ladite chambre (2), ledit piston (51) étant fixé à l'extrémité libre d'une tige (52) d'un vérin (54).

11. Dispositif selon la revendication 10, caractérisé en ce que la section active du piston (51) est inférieure à la section active du vérin (54).

12. Dispositif selon la revendication 3, caractérisé en ce que les moyens (70) de récupération des volumes successifs du produit stérilisé sont formés par une vanne à tiroir (73) de vidange comportant, à sa partie inférieure, un orifice transversal (74) et, à sa partie supérieure, un canal de circulation (75) du produit stérilisé communiquant avec un réservoir (71) de récupération stérile par un tuyau télescopique (76), ladite vanne à tiroir (76) de vidange étant déplaçable verticalement par un organe de commande (78) entre une première position mettant en communication l'orifice transversal (74) avec la chambre (2) et une seconde position mettant en communication le canal de circulation (75) avec ladite chambre (2).

13. Dispositif selon la revendication 12, caractérisé en ce que la vanne à tiroir (73) de vidange est formée par une plaque de section rectangulaire.

14. Dispositif selon les revendications 12 et 13, caractérisé en ce que l'organe de commande du déplacement de la vanne à tiroir (73) de vidange est constitué par au moins un vérin (78).

15. Dispositif selon la revendication 3, caractérisé en ce que les moyens de séparation du volume du produit stérilisé avec le volume du produit à stérilisé sont formés par des séparateurs cylindriques (3) de diamètre correspondant au diamètre de la chambre (2), destinés à être déplacés dans la chambre (2) entre chaque volume de produit et comportant des organes d'étanchéité avec la paroi de ladite chambre (2).

16. Dispositif selon les revendications 3 et 10, caractérisé en ce que les moyens de stérilisation de la première extrémité (2a) de la chambre (2) sont formés par un canal (25) ménagé dans le piston (51) et débouchant au niveau de l'extrémité libre dudit piston (51), ledit canal (25) étant relié par un orifice (26) à des moyens d'injection de vapeur d'eau.

17. Dispositif selon l'une quelconque des revendications 3 à 16, caractérisé en ce que les moyens (90) de transfert des séparateurs cylindriques (3) sont formés par un tuyau de retour (91) s'étendant d'une extrémité à l'autre du tube (1) et comportant à une de ses extrémités le sas (92) relié à la seconde extrémité (2b) de la chambre (2) par la vanne à tiroir (73) de vidange et à l'autre de ses extrémités le sas (110) relié à la première extrémité (2a) de la chambre (2) par la vanne à tiroir (13) de remplissage.

18. Dispositif selon la revendication 17, caractérisé en ce que le tuyau de retour (91) comprend à son extrémité (91a) située à proximité de l'extrémité (2b) de sortie de la chambre de traitement (2), un organe (93) d'introduction des séparateurs cylindriques (3) dans le tuyau de retour (91), des moyens (98) de propulsion des séparateurs cylindriques (3) dans ledit tuyau de retour (91) et des moyens (99) de nettoyage desdits séparateurs cylindriques (3).

19. Dispositif selon la revendication 18, caractérisé en ce que l'organe d'introduction des séparateurs cylindriques (3) dans le tuyau de retour (91) est constitué par un piston (94) d'un vérin (96).

20. Dispositif selon la revendication 18, caractérisé en ce que les moyens (98) de propulsion des séparateurs cylindriques (3) dans le tuyau de retour (91) sont formés par un fluide sous pression injecté dans ledit tuyau de retour (91).

21. Dispositif selon l'une quelconque des revendications 3 à 20, caractérisé en ce que les moyens de stérilisation du sas (92) de récupération sont formés par un orifice (85) ménagé dans une paroi dudit sas et relié à des moyens d'injection de vapeur d'eau.
